# EUROPEAN PATENT APPLICATION

(11) **EP 2 881 048 A1**
(43) Date of publication of application: **10.06.2015**
(21) Application number: 13825274.7
(22) Date of filing: 26.07.2013
(51) Int. Cl.: A61B 17/28

(54) **MEDICAL MANIPULATOR**

(30) Priority: 31.07.2012 JP 2012169667
(71) Applicant: Olympus Corporation, Tokyo 151-0072 (JP)
(72) Inventor: KOMURO, Takahiro, Tokyo 151-0072 (JP); IIDA, Masatoshi, Tokyo 151-0072 (JP)
(74) Representative: Schicker, Silvia
(86) International application number: PCT/JP2013/070349
(87) International publication number: WO 2014/021222

(57) **Abstract**

A medical manipulator (1) comprises an insertion part (50), an arm part (65), a master input part (2), and a control part (8). The insertion part (50) further comprises a bending part (52) in a portion of the insertion part (50). The insertion part (50) is provided with the arm part (65) on the distal end of the insertion part (50). The arm part (65) further comprises an arm (61) and a treatment part (73). An operator (Op) makes a manipulation with the master input part (2). The control part (8) either bends the bending part (52) or operates the arm part (65), in response to an input from the master input part (2). The master input part (2) further comprises fool switches (23) for the operator (Op) to select a mode. When the operator (Op) inserts the insertion part (50) into the body of a patient (P) and selects an insertion mode. In this mode, the arm part (65) is locked, although the bend part (52) is capable of being manipulated. When the operator (Op) carries out a procedure within the body of the patient (P), the operator (Op) selects a treatment mode. In this mode, the bending part (52) is locked, and the arm part (65) is capable of being manipulated.

## Description

### Technical Field

The present invention relates to a medical manipulator. More particularly, the present invention relates to a medical manipulator in which a part inserted into the body of a patient can be bent.

Priority is claimed on Japanese Patent Application No. 2012-169667, filed July 31, 2012, the content of which is incorporated herein by reference.

### Background Art

In the related art, a medical manipulator in which a part to be inserted into the body of a patient or the like who undergoes a procedure is bendable is known (for example, refer to Patent Literature 1). In such a medical manipulator, a desired procedure is performed by manipulating a treatment part provided on a distal end part of the medical manipulator with a proximal manipulation part while observing a region where the procedure is performed with observation means, such as an endoscope.

The medical manipulator of Patent Literature 1 includes a switching tube part in which a number of switching tubes that function as joints are arranged in a part to be inserted into the body. The manipulation part is provided with a dial, and can be switched between a rigid state where a bent state of the switching tube part is held

(locked), and a flexible state where the switching tubes move freely by manipulating the dial.

Accordingly, insertion is easy and patient discomfort is minimized by switching the medical manipulator into the flexible state when the medical manipulator is being inserted into the body, and a stable state while a procedure is being formed can be obtained by switching the medical manipulator into the rigid state.

### Citation List

### Patent Literature

[Patent Literature 1] Japanese Unexamined Patent Application, First Publication No. 2008-289556

### Summary of Invention

### Problem to be Solved by Invention

However, although the state of the switching tube part can be switched to the rigid and flexible states in the medical manipulator of Patent Literature 1, the treatment part is the same in any state. Accordingly, since the dimensions of a relatively rigid part in the distal end part of the medical manipulator do not change, there is a problem in that insertion may not be easy depending on the type or structure of the treatment part.

The invention has been made in view of the above situation, and an object thereof is to provide a medical manipulator that can favorably perform insertion into a body irrespective of the type or structure of a treatment part.

### Means for Solving Problem

According to a first aspect of the invention, a medical manipulator includes an insertion part which includes an external cylindrical tube that has a bendingly manipulable bending part and that has flexibility; a manipulation part which performs a manipulation input for manipulating the insertion part; an arm part which is provided on a distal end part of the insertion part and has a bendingly manipulable arm and a treatment part that performs a procedure; a mode-input part which is provided on the manipulation part and is capable of inputting a mode that is selected among a plurality of modes that are combinations of states of the bending part and the arm part; and a control part that selectively controls the bending part and the arm part such that the bending part and the arm part are controlled to enter one of two states consisting of a locked state in which the bending part and the arm part are locked in a predetermined state in which the manipulation input is not accepted, and a non-locked state, on the basis of the manipulation input and a mode input to the mode-input part.

According to a second aspect of the present invention, in the medical manipulator according to the first aspect, at least one of the locked states of the arm part may be a retracted state in which the arm part is arranged within a width of the external cylindrical tube.

According to a third aspect of the present invention, in the medical manipulator according to the second aspect, at least one of the non-locked states may be a manipulable state in which the bending part or the arm part is operated according to the manipulation input.

According to a fourth aspect of the present invention, in the medical manipulator according to the third aspect, at least one of the plurality of modes may be an insertion mode suitable for inserting the insertion part into a body, and in the insertion mode, the bending part may be brought into the manipulable state and the arm part may be brought into the retracted state.

According to a fifth aspect of the present invention, in the medical manipulator according to the fourth aspect, in this case, a rigid length of the insertion part on a distal end side in the retracted state may be smaller than a maximum value of a rigid length in a procedure.

According to a sixth aspect of the present invention, in the medical manipulator according to the fourth aspect, at least one of the plurality of modes may be a treatment mode suitable for performing a procedure using the treatment part, and in the treatment mode, the bending part may be brought into the locked state and the arm part may be brought into the manipulable state.

According to a seventh aspect of the present invention, in the medical manipulator according to the fourth aspect, at least one of the plurality of modes may be a treatment mode suitable for performing a procedure using the treatment part, and in the treatment mode, the bending part and the arm part may be brought into the manipulable state.

According to an eighth aspect of the present invention, in the medical manipulator according to any one of the fourth aspect to the seventh aspect, at least one of the plurality of modes may be an urgent extraction mode suitable for rapidly extracting the insertion part out of the body, and in the urgent extraction mode, the bending part and the arm part may be brought into a free state which is one of the non-locked states and in which an operation in response to the manipulation input is not performed and deformation by an external force is allowed.

According to a ninth aspect of the present invention, in the medical manipulator according to the sixth aspect or the seventh aspect, the arm part may have the arm fixed to the distal end part of the external cylindrical tube, and a treatment tool having the treatment part on a distal end part of the treatment part and being inserted through the external cylindrical tube and the arm, and a protruding amount of the treatment part from the arm in the insertion mode may be smaller than a protruding amount of the treatment part from the arm in the treatment mode.

### Advantageous Effects of Invention

According to the medical manipulator in the above respective aspects, insertion of the treatment part into a body can be favorably performed irrespective of the type or structure of the treatment part.

### Brief Description of Drawings

FIG. 1 is a view showing an overall configuration of a master slave system that is a medical manipulator according to a first embodiment of the invention.
FIG. 2 is an enlarged view showing a distal end side of an insertion part in the master slave system.
FIG. 3 is a functional block diagram of a portion of the master slave system.
FIG. 4 is a transition diagram in respective modes of the insertion part in the master slave system.
FIG. 5 is a view showing an example of respective processes and applied modes when the master slave system is used.
FIG. 6 is a view showing the setting contents of respective parts of the master slave system in the respective modes.
FIG. 7A is a view showing the shape of an arm part of the master slave system in a retracted state.
FIG. 7B is a view showing an example of the state of the arm part in a procedure.
FIG. 8A is a perspective view showing a distal end part of the insertion part in a modification example of the invention.
FIG. 8B is a view showing a retracted state of the insertion part.
FIG. 9 is an enlarged view showing an insertion part distal end side of a master slave system according to a second embodiment of the invention.
FIG. 10 is a view showing the setting contents of respective parts of the master slave system in the respective modes.

### Description of Embodiments

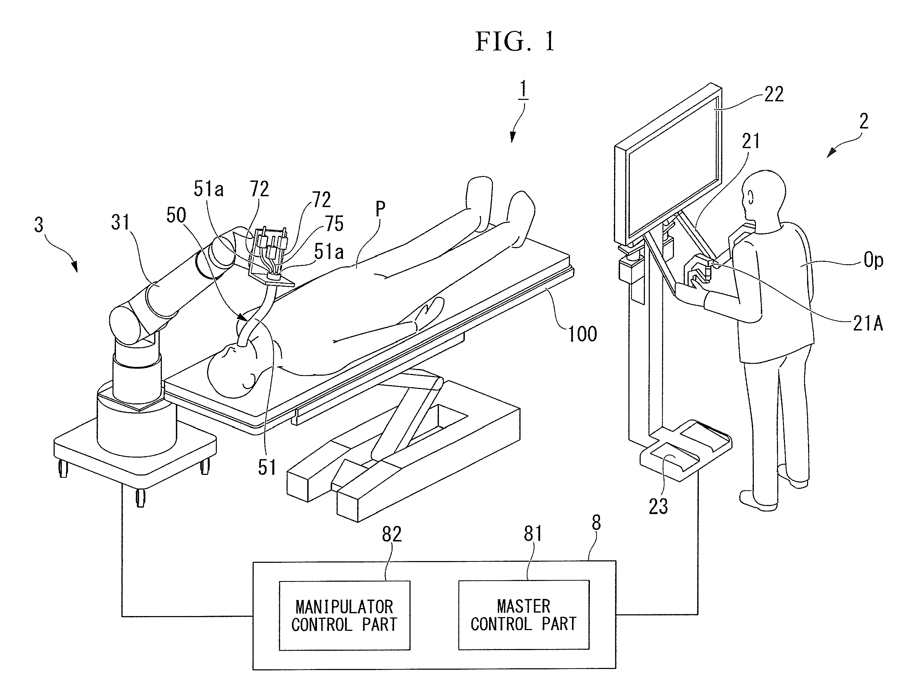

A first embodiment of the invention will be described with reference to FIGS. 1 to 7B. FIG. 1 is a view showing an overall configuration of a master slave system that is a medical manipulator according to the first embodiment of the invention. The master slave system 1 includes a master input part (manipulation part) 2 having a master arm 21 and being provided for performing a manipulation input, and a slave manipulator 3 having a slave arm 31. The master slave system 1 remotely controls the slave arm 31 and the insertion part (to be described below) so that the slave arm is made to follow the manipulation of the master arm 21 by a surgeon (operator) Op. A manipulation command via the master arm 21 is transmitted to a master control part 81 of a control part 8, is appropriately subjected to conversion processing if necessary, and then is input to a manipulator control part 82. Thereafter, an operation signal is sent from the manipulator control part 82 to the slave manipulator 3, and the slave arm 31 and the insertion part are operated.

As shown in FIG. 1, the slave manipulator 3 is installed in a surgical table 100 on which a patient P is placed. Since the slave arm 31 is configured to have a plurality of multi-degree-of-freedom joints, a multiaxial operation is possible. Each multi-degree-of-freedom joint is individually driven by a power part that is not shown. As the power part, for example, a motor (servo motor) having a servo mechanism including an incremental encoder, a speed reducer, or the like, can be used.

An insertion part 50 to be inserted into the body of the patient P is attached to a distal end part of the slave arm 31.

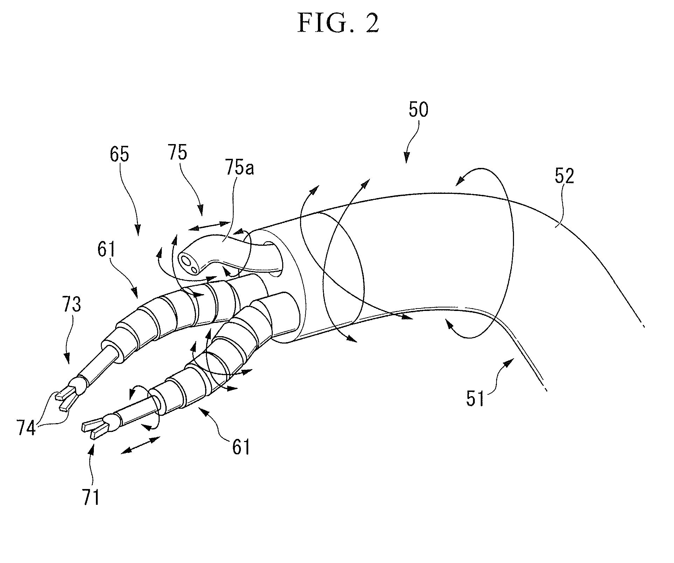

FIG. 2 is an enlarged view showing a distal end part of the insertion part 50. The insertion part 50 has an external cylindrical tube 51 that is elongated and has flexibility, and two arms 61 that are attached to the distal end part of the external cylindrical tube 51. The range of a predetermined length on a distal end side of the external cylindrical tube 51 is a bending part 52 that has a well-known structure in which a plurality of joint rings, bending pieces, or the like are arranged. The bending part can be bent in biaxial directions orthogonal to an axis of the external cylindrical tube 51 by advancing and retracting a manipulating member (not shown), such as a wire connected to a joint ring or the like closest to the distal end side, with respect to the external cylindrical tube 51.

The external cylindrical tube 51 is attached so as to be rotatable around its own axis with respect to the slave arm 31. A manipulating member for rotating the external cylindrical tube 51 and the manipulating member (not shown) for manipulating the bending part 52 are pulled out from a proximal end side of the external cylindrical tube 51 and are fixed to pulleys (not shown), respectively, and a shaft of each pulley is coupled to a drive shaft (not shown) that is provided on the slave arm 31. Each drive shaft is provided with a driving mechanism (not shown) that has the same configuration as the above-described power part, and the bending manipulation and rotation manipulation of the external cylindrical tube 51 can be performed by rotating the drive shafts with the driving mechanisms. Hereinafter, the drive shafts and the driving mechanisms may be generically referred to as an "external cylindrical tube drive part".

The two arms 61 are attached to the distal end of the external cylindrical tube 51. Each arm 61 has the same bending structure as the bending part 52, and can be bent in biaxial directions orthogonal to an axis of each arm 61. Additionally, each arm 61 is formed in a tubular shape having an inner cavity, and the inner cavity communicates with an inner cavity of a treatment tool channel (not shown) provided in the external cylindrical tube 51.

An arm manipulating member for bendingly manipulating each arm 61 is coupled to a drive shaft for an arm provided on the slave arm, in the same manner as the manipulating member of the external cylindrical tube, and each arm 61 can be bent with the driving mechanism for an arm. Hereinafter, the drive shafts and the driving mechanisms may be generically referred to as an "arm drive part".

A treatment tool 71 for performing a treatment is inserted through each arm 61. The treatment tool 71 includes an elongated sheath part 72 (refer to FIG. 1) having flexibility, and a treatment part 73 provided on the distal end of the sheath part 72. In FIG. 2, a treatment part having a pair of forceps members 74 that are opened and closed is shown as an example of the treatment part 73. However, in the master slave system 1, as the treatment tool, a plurality of types of treatment tools having different treatment parts corresponding to various procedures, such as a high-frequency knife and a snare loop, are prepared, and are appropriately replaced and used depending on procedures or the like. Accordingly, there is also a treatment part that is not driven depending on the configuration of the treatment part. The treatment tool 71 is inserted into the treatment tool channel of the external cylindrical tube 51 from a forceps port 51a provided in a proximal end part of the external cylindrical tube 51, and is inserted through the inside of the arm 61 that communicates with the treatment tool channel through the inside of the treatment tool channel, and the treatment part 73 protrudes from a distal end opening of the arm 61.

A treatment tool manipulating member (not shown), such as a wire, is attached to each forceps member 74, and the treatment part 73 can be driven by advancing and retracting the treatment tool manipulating member with respect to the treatment part 73. The treatment tool manipulating member is coupled to a drive shaft for a treatment tool provided on the slave arm 31 in the same aspect as the manipulating member of the external cylindrical tube and the arm manipulating member, and the treatment part 73 is driven by the driving mechanism for a treatment tool. Additionally, the treatment tool 71 is advanceable/retractable and rotatable with respect to the external cylindrical tube 51 and the arm 61. The protruding length of the treatment part 73 from the distal end opening of the arm 61 can be adjusted by advancing and retracting the treatment tool 71, and the opening/closing direction of the treatment part 73 can be adjusted in a suitable state with respect to a procedure by rotating the treatment tool 71. A manipulating member for advancing/retracting and rotating the treatment tool 71 is also connected to a drive shaft and a driving mechanism that are respectively provided on the slave arm 31, similar to the above-described respective manipulating members. Hereinafter, respective drive shafts and respective driving mechanisms for driving respective parts of the treatment tool 71 may be generically referred to as a "treatment tool drive part".

An arm part 65 for performing a procedure on a target region is configured by the two arms 61, and the treatment tool 71 that has the treatment part 73 and is inserted through each arm 61. By means of the above-described configuration, in the arm part 65, the bending of each arm 61 in the biaxial directions orthogonal to the axis of the arm and the advance/retraction and rotation of the treatment tool 71 are allowed. The surgeon Op can appropriately perform these manipulations via a master input part 2, thereby bringing the position and posture of the treatment part 73 into a desired state.

As shown in FIG. 2, observation means 75 for acquiring an image in front of the external cylindrical tube 51 protrudes from the distal end of the external cylindrical tube 51, in addition to the arms 61. Since the observation means 75 is inserted through a channel for observation means provided in the external cylindrical tube 51, the observation means is advanceable/retractable and rotatable with respect to the external cylindrical tube 51. Moreover, the observation means has a bending part 75a on a distal end side thereof, and is bendable. As such observation means 75, various well-known endoscopes or the like can be suitably used. Since a manipulating member for performing various manipulations of the observation means 75 is also coupled to the slave arm 31 similar to the external cylindrical tube 51 and the arm part 65, the manipulating member can be manipulated via the master input part 2. Hereinafter, respective drive shafts and respective driving mechanisms for driving respective parts of the observation means 75 may be generically referred to as an "observation means drive part".

The master input part 2, as shown in FIG. 1, includes a plurality of master arms 21 to be manipulated by the surgeon Op, and a display part 22 on which an image acquired by the above-described observation means 75 is displayed. Each master arm 21 has a well-known configuration in which multiaxial operation is allowed, and includes a gripping part 21A as a manipulation part, which is gripped by the surgeon Op and issues a manipulation command, on the distal end side near the surgeon Op. Moreover, the master input part 2 is provided with a foot switch (mode-input part) 23 for selecting and inputting the modes of the insertion part 50 to be described below.

The configuration of the mode-input part is not limited to the foot switch and may be, for example, buttons provided on the gripping part 21 A, a touch panel displayed on the display part 22, or the like, and there is no particular limitation to a specific configuration. Additionally, multiple configurations may be appropriately combined.

In addition, the modes of the insertion part 50 will be described below in detail.

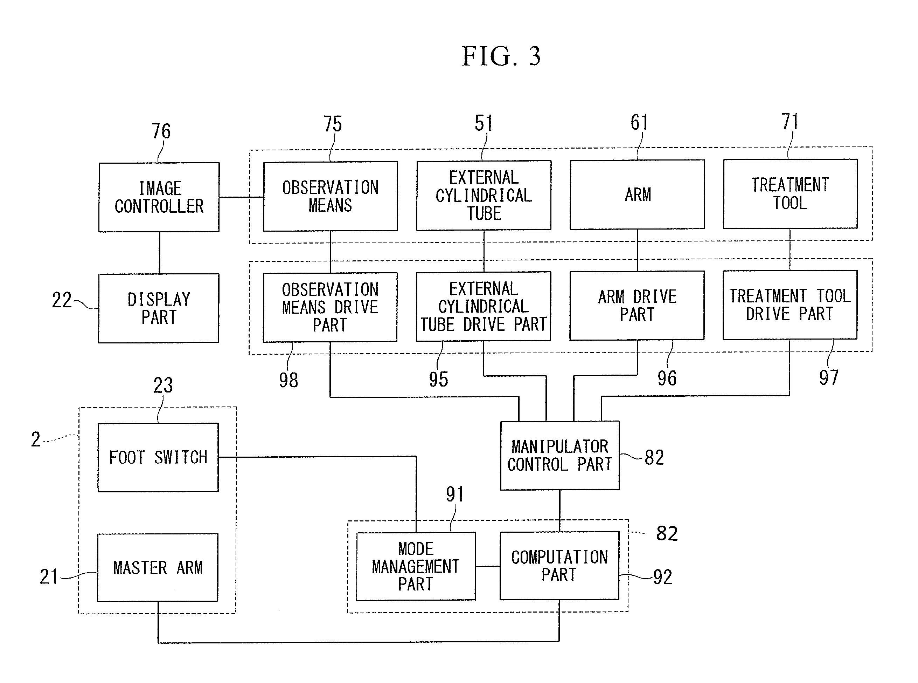

FIG. 3 is a functional block diagram of a portion of the master slave system 1. The master control part 81 includes a mode management part 91 connected to the foot switch 23, and a computation part (control part) 92 connected to the master arms 21 and the mode management part 91. A manipulation input regarding a mode of the insertion part 50 via the foot switch 23 is sent to the mode management part 91, and a signal indicating the selected mode is sent from the mode management part 91 to the computation part 92.

The computation part 92 generates signals for operating respective parts of the slave manipulator 3 on the basis of the manipulation inputs from the master arms 21 and the signal received from the mode management part 91, sends the signals to the manipulator control part 82, and controls the operation of respective parts of the insertion part 50.

The manipulator control part 82 is connected to the external cylindrical tube drive part 95 that drives the external cylindrical tube 51, the arm drive part 96 that drives the respective arms 61, the treatment tool drive part 97 that drives the respective treatment tools 71, and the observation means drive part 98 that drives the observation means. Detection means, such as encoders, which detects the amounts of displacement of the manipulating members of the coupled respective parts, is attached to the drive shafts of the respective drive parts 95, 96, 97, and 98, signals indicating the amounts of displacement are sent from the respective drive parts to the manipulator control part 82, and various manipulation amounts, such as the bending amounts or protruding amounts of the respective parts, are recognized. The manipulator control part 82 generates driving signals on the basis of these manipulation amounts and the signals received from the computation part 92, and sends the driving signals to the respective drive parts. Accordingly, the insertion part 50 and the respective parts inserted through the insertion part 50 are operated.

The observation means 75 is connected to the image controller 76, an image of a surgical field acquired by the observation means 75 is sent to the display part 22 via the image controller 76, and the image is displayed on the display part 22.

The operation when the master slave system 1 is configured as mentioned above will be described.

The surgeon Op first inserts the insertion part 50 into the body from a natural opening of the Patient P. Although FIG. 1 illustrates an example where the insertion part is inserted from a mouth, the insertion part 50 may be inserted from other natural openings, such as the anus, instead of this. The surgeon Op moves the distal end of the insertion part 50 to a target region of a procedure, while confirming the image of the observation means 75 with the display part 22.

Next, the surgeon Op selects a treatment tool in accordance with a procedure to be performed, mounts the selected treatment tool on the slave arm 31 to insert the mounting tool into the forceps port 51a of the insertion part 50, and makes the treatment part 73 protrude from an arm 61. The protruding amount in this case is a predetermined initial protruding amount, and may be, for example, a maximum protruding amount such that an engaging part provided on the treatment tool 71 is engaged with an engaged part provided on the arm and the treatment part 73 cannot protrude any more.

Thereafter, the surgeon Op manipulates the two arm parts 65 while viewing the image of the observation means 75, and performs a desired procedure on a target region. If necessary, the treatment tool may be replaced, or the distal end part of the insertion part 50 may be moved to other target regions so as to perform a procedure.

If all procedures are finished, the insertion part 50 is extracted from the patient P, and the surgeon Op ends a series of operations.

Although the above is the outline of the operation when the master slave system 1 of the present embodiment is used, a plurality of modes configured by the combination of the states of the respective parts of the insertion part 50 are set in the master slave system 1. Thus, the above-described respective operations can be suitably performed by selecting appropriate modes depending on the situation.

Operation states, such as the bending of the bending part 52 of the external cylindrical tube 51, the bending of the respective arms 61, the protruding amount and opening/closing of the treatment part 73 in the treatment tool 71, and the protruding amount and the bending part 75a of the observation means 75 are set in the respective modes. Respective portions are set to any one of three types roughly including a "locked state" where a predetermined state (shape) is held (locked) and a manipulation input from the master arm 21 is not received, a "manipulable state" where an operation is made according to the manipulation from the master arm 21, and a "free state" where the manipulation input of the master arm is not received and deformation is made due to an external force according to its own degree of freedom.

Methods for realizing the locked state are not particularly limited, and various well-known methods can be used. For example, a manipulating member may be held at a predetermined position by the servo control of a driving mechanism, or the manipulating member may be held by a brake or the like installed at the driving mechanism. Additionally, the insertion part 50 may be held at a predetermined position by the servo control of the driving mechanism (each drive part), or may be held by the brake or the like installed at the driving mechanism. Additionally, when the insertion part 50 is driven by a wire, the wire may be fixed so as not to be moved.

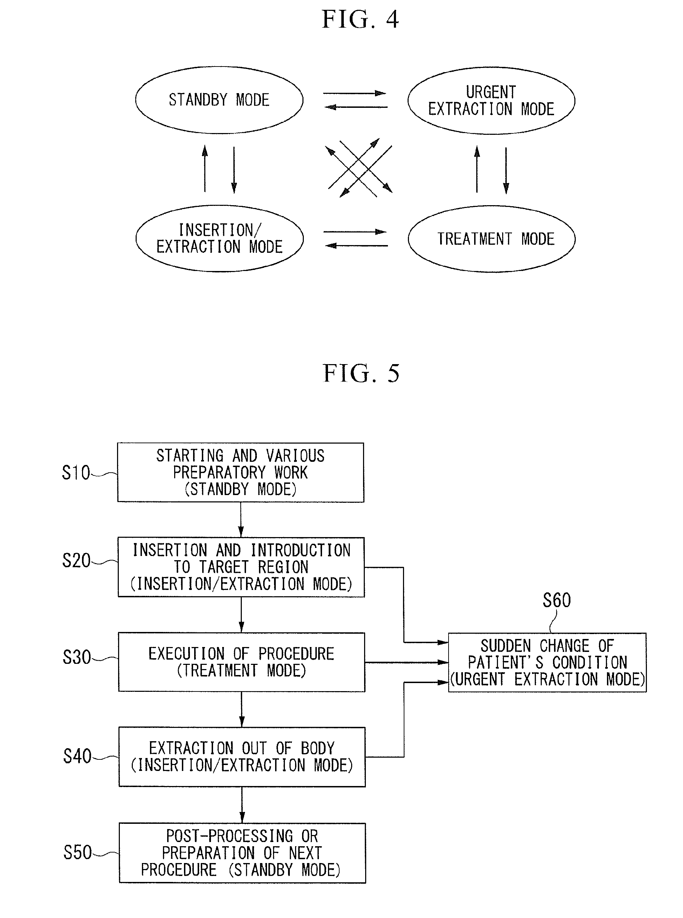

The respective modes of the master slave system 1 and a transition diagram thereof are shown in FIG. 4. In the master slave system 1, four modes, that is, a standby mode, an insertion/extraction mode (insertion mode), a treatment mode, and an urgent extraction mode, are prepared, and direct transition without transition via other modes is allowed from the respective modes to the other three modes.

Next, an example of the correspondence of the modes in respective processes of the operation in the above-described use and the specific setting contents of the respective modes will be described with reference to FIGS. 5 to 7B.

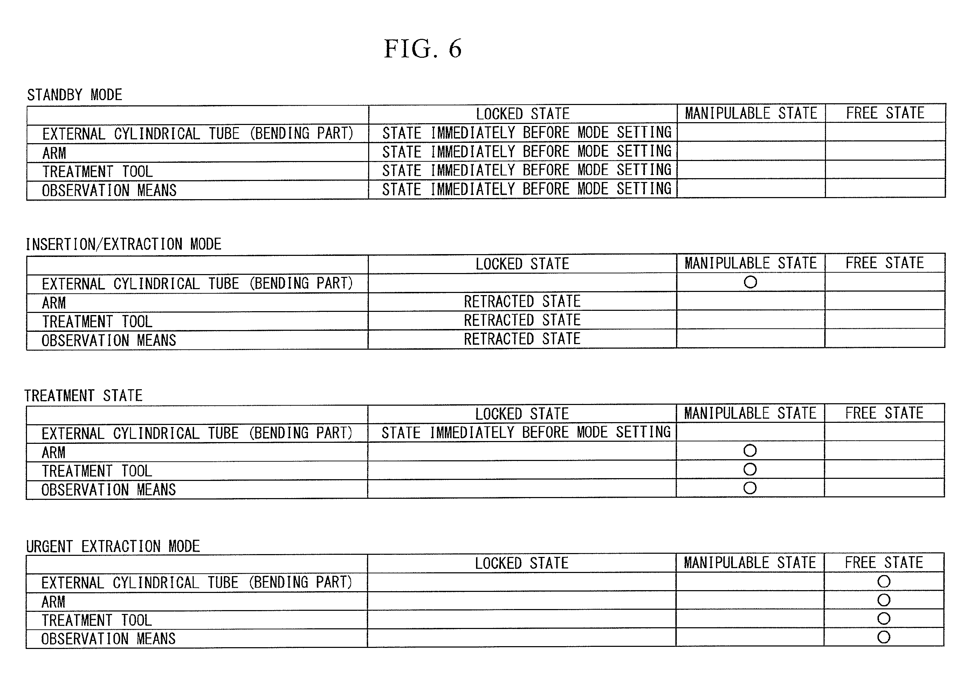

In Step S10 of FIG. 5, the master slave system 1 is started and various kinds of preparatory work, such as initialization, are performed. Here, the standby mode is applied as a user's input or the initial setting of the system. As shown in FIG. 6, in the standby mode, all parts are set to the locked state, and are held in a shape immediately before mode setting.

In Step S20 where the insertion part 50 is inserted into the body of a patient from a natural opening and is introduced to a target region, the insertion/extraction mode is applied. As shown in FIG. 6, in the insertion/extraction mode, only the bending part 52 of the external cylindrical tube 51 that requires a bending manipulation is brought into a manipulable state in insertion and introduction, and the other parts are set to the locked state. Here, unlike the standby mode, the respective parts are locked in a predetermined shape that is defined as a "retracted state".

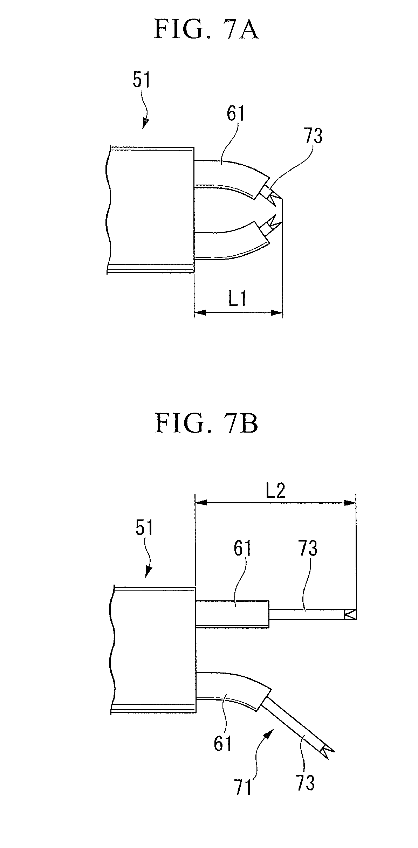

An example of a shape in the retracted state of the arms 61 and the treatment tools 71 is shown in FIG. 7A. The two arms 61 are locked in a state where each arm is bent toward the other arm, and each treatment tool 71 is locked in a state where the protruding length of the treatment part 73 from the arm 61 is shorter than it is when a procedure is performed. The sum L1 (first rigid length) of the length of each arm 61 protruding from the distal end part of the external cylindrical tube 51 and the length of the treatment part 73 protruding from the arm 61 (hereinafter referred to as a "rigid length"), as the arm 61 and the treatment tool 71 are locked to the retracted state, becomes shorter than the maximum value L2 of a rigid length (second rigid length) that is the sum of the length of the arm 61 protruding from the distal end part of the external cylindrical tube 51 and the length of the treatment part 73 protruding from the arm 61, during a procedure, as shown in FIG. 7B. Simultaneously, the arm 61 and the treatment tool 71 are located within the width of the external cylindrical tube 51, and, the parts that protrude out of the width of the external cylindrical tube 51 are eliminated. Here, the rigid length refers to the total length of the length of the arm 61 and the length of the treatment part 73 that protrude to the outside from the distal end part of the external cylindrical tube 51, and is the length of the part that is movable during treatment.

In the retracted state, the observation means 75 is retracted and locked to a position where the distal end thereof does not protrude from the external cylindrical tube 51.

In Step S30 that executes a procedure on the target region, the treatment mode is applied. In the treatment mode, as shown in FIG. 6, only the external cylindrical tube 51 is locked in a state immediately before mode setting, and the other parts are set to a manipulable state. Accordingly, since the minute manipulation of the arms 61, the treatment tools 71, the observation means 75, or the like can be performed in a state where the basic positions of the arms 61 and the observation means 75 are fixed, the surgeon Op can stably perform a procedure.

In Step S40 where the insertion part 50 is extracted to the outside of the body of the patient P after the end of the procedure, the insertion/extraction mode is applied. Thereafter, in Step S50 where preparation of the next procedure and post-processing after use are performed, the standby mode is applied.

When the insertion part 50 is within the body of the patient P and it is necessary to rapidly move the insertion part 50 to the outside of the body due to the patient's condition having changed suddenly or the like (Step S60 shown in FIG. 5), the urgent extraction mode is applied. In the urgent extraction mode, as shown in FIG. 6, all the parts are set in the free state. Accordingly, even if the surgeon or the like rapidly pulls out the insertion part 50, the respective parts of the insertion part 50 can be smoothly deformed in accordance with the shape of a tissue or the like within the body, thereby minimizing a discomfort caused to the patient P.

In a medical manipulator including a bendable arm, the arm is bent in various directions during a procedure. As a result, as shown in FIG. 7B, portions of the arms 61, the treatment parts 73 of the treatment tools 71 inserted through the arms 61, or the like may protrude out of the range of the width of the external cylindrical tube 51. If the insertion or extraction of the insertion part is performed in such a state, a protruding part may be caught in the tissue in the body or the like, or may damage the tissue or the like.

By lengthening the length of the arm in the medical manipulator, a range where treatment is allowed can be increased, whereas the dimension of the arm in an axis direction becomes long. If the dimension of the arm in the axis direction becomes long, the bending radius of the insertion part on the distal end side increases. As a result, there is a problem in that it is difficult for the insertion part to pass through a region, such as the large intestine, which is strong against flexure.

According to the master slave system 1 of the present embodiment, in the plurality of modes provided by combining the states of the respective parts of the insertion part 50, the computation part 92 selectively controls the bending part 52 and the arm part 65 to the locked state where the manipulation input is not received and the shape is held, and a non-locked state, respectively, on the basis of a mode when an input is made via the foot switch 23.

In the insertion/extraction mode that is one of the modes, the arm part 65 including the bendable arms 61 and the treatment tools 71 having the treatment parts 73 is locked to the retracted state. As a result, the arm part 65 falls within the range of the width of the external cylindrical tube 51 regardless of the specific configuration of the treatment parts 73, and the insertion and extraction of the insertion part can be smoothly performed.

Additionally, since the rigid length L1 in the retracted state becomes shorter than the maximum value L2 of the rigid length when a procedure is performed using the treatment parts 73, further suppression of a catch during insertion or extraction can be expected.

Additionally, in the treatment mode that is one of the modes, since the bending part 52 of the external cylindrical tube 51 is set to the locked state, the surgeon does not need to perform a manipulation for holding the shape of the external cylindrical tube, and can proceed with a procedure while concentrating on the manipulation of the arm part or the like. As a result, a user-friendly medical manipulator can be provided.

Moreover, since the arm part 65 is constituted of the arms 61 that are fixed to the external cylindrical tube 51, and the treatment tools 71 that are inserted through the external cylindrical tube 51 and the arms 61 and have the treatment parts 73, the rigid length in the retracted state can be set to be shorter by changing the protruding length of the treatment parts from the arms.

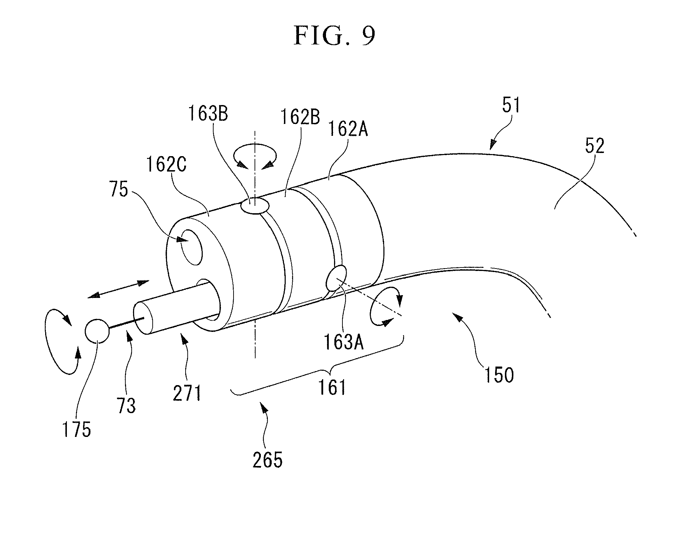

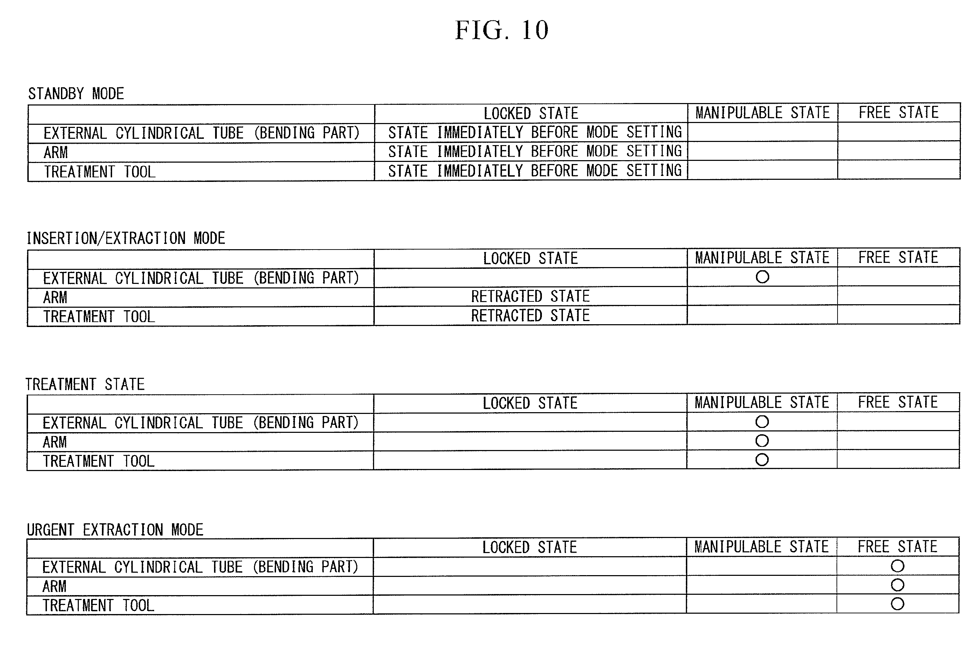

Subsequently, a second embodiment of the invention will be described with reference to FIGS. 9 and 10. In addition, in the following description, components common to those already described will be designated by the same reference numerals, and a duplicate description will be omitted.

FIG. 9 is an enlarged view showing a distal end side of an insertion part 150 in a master slave system of the present embodiment. In the insertion part 150, an arm 161 having almost the same external diameter as the external cylindrical tube 51 is provided on a distal end of the external cylindrical tube 51. The arm 161 has three annular members 162A, 162B, and 162C arranged side by side in an axis direction. The annular members 162A and 162B are coupled together so as to be relatively rotatable in a rotary shaft portion 163A. The annular members 162B and 162C are coupled together so as to be relatively rotatable in a rotary shaft portion 163B. Since the axis of the rotary shaft portion 163A and the axis of the rotary shaft portion 163B are shifted in phase by about 90 degrees in a circumferential direction of the arm 161, the arm 161 can be bent in biaxial directions orthogonal to an axis thereof as the respective annular members 162A, 162B, and 162C rotate around the respective rotary shaft portions 163A and 163B. As a mechanism for driving the arm 161, for example, substantially the same mechanism as that of the first embodiment can be used.

A treatment tool 271 having a knife part 175, which applies a high-frequency current, at the treatment part 73 is shown as an example of the treatment tool in FIG. 9. The arm part 265 having the arm 161, the treatment tool 271, and the like is the same as that of the first embodiment in that a plurality of types of treatment tools having different treatment parts are prepared, and are appropriately replaced and used depending on procedure or the like.

The observation means 75 is fixed to a distal end part of the arm 161, and is not advanced and retracted with respect to the arm 161.

FIG. 10 is a view showing the setting contents in the respective modes in the master slave system of the present embodiment. As described above, since the observation means 75 is fixed to the arm 161, the observation means is not described. Additionally, the present embodiment is different from the first embodiment in that the bending part 52 of the external cylindrical tube 51 is brought into a manipulable state in the treatment mode. The arm 161 has a structure in which the arm has higher flexure (smaller bending radius) than the bending part 52. Therefore, the present embodiment is suitable for treatment in a narrow range. Accordingly, in the treatment mode, a treatment can be performed by the arm 161 by moving to a place where treatment is desired, using the bending part 52.

While the respective embodiments of the invention have been described above, the technical scope of the invention is not limited to the above embodiments. Combinations of constituent elements can be changed, various alternations can be added to the respective constituent elements, or omissions can be made, without departing from the concept of the invention.

For example, in the medical manipulator of the invention, the observation means is not necessarily provided on the insertion part. That is, the observation means may be attached to another slave arm for the observation means and introduced into the vicinity of a target region, or a port may be provided in an abdominal wall and parenterally introduced, using a laparoscope as the observation means. In this case, it is needless to say that that it is not necessary to set the state of the observation means in the respective modes.

Additionally, the number of arm parts is also not particularly limited, and may be one or may be three or more.

Moreover, the specific shape in the retracted state is also not particularly limited if the shape is a form that falls within the range of the width of the external cylindrical tube. Here, if the form in the retracted state is set so that the distal ends of the plurality of arm parts 65 are gathered at the same point as in the above-described embodiments, the maximum dimensions of the insertion part 50 in the width direction in the retracted state become smaller approaching the distal ends. Therefore, insertion or extraction can be more suitably performed, which is preferable.

Moreover, in the medical manipulator of the invention, the configuration of the arm part is not limited to the above-described example, and can be variously changed. The shape in the retracted state can also be variously set according to respective configurations.

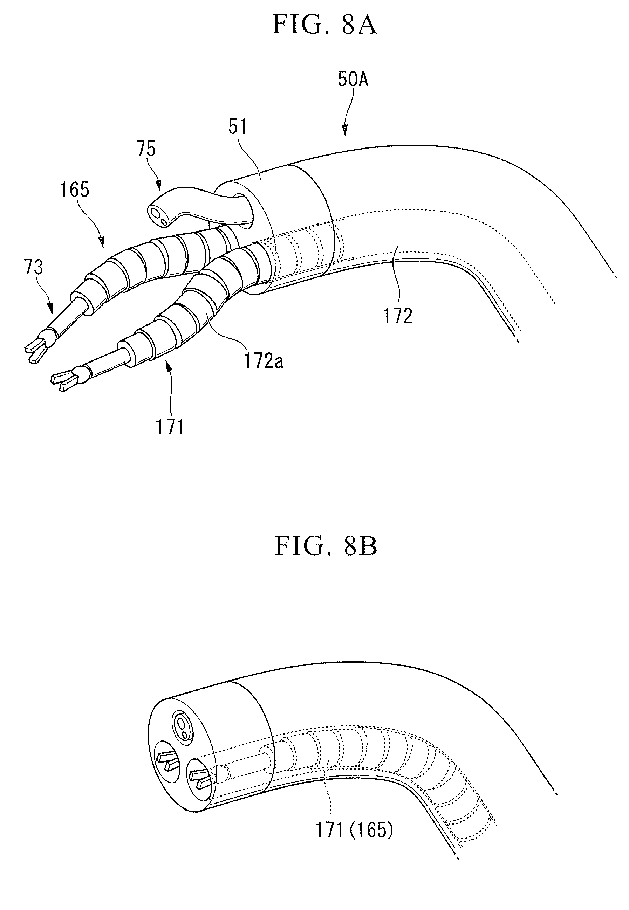

For example, in an insertion part 50A of a modification example shown in FIG. 8A, the arms 61 are not provided on the external cylindrical tube 51. Instead, an arm part 165 is provided by inserting treatment tools 171 including bendingly manipulable bending parts 172a on the distal end side of the sheath part 172 through treatment tool channels of the external cylindrical tube 51 and making regions on the distal end side including the bending parts 172a protrude from the external cylindrical tube 51. Although the insertion part 50A may also possibly take almost the same retracted shape as that of the above-described embodiment, since the treatment tools 171 are advanceable and retractable with respect to the external cylindrical tube 51, the arm part 165 can be completely housed within the external cylindrical tube 51 and brought into the retracted state by retracting the treatment tools 171 as shown in FIG. 8B. In this case, the insertion and extraction of the insertion part can be more easily performed.

In addition, a treatment tool having a joint on a distal end side thereof may be inserted through an arm fixed to the external cylindrical tube.

Moreover, other modes may be further set in addition to the above-described modes.

Additionally, the medical manipulator of the invention is not limited to a configuration in which the insertion part as described above is remotely controlled by the manipulation part, and may have a configuration in which the manipulation part and the insertion part are integrally provided.

### Industrial Applicability

According to the medical manipulators in the above respective embodiments, insertion of the treatment part into a living body can be favorably performed irrespective of the type or structure of a treatment part.

### Reference Signs List

1: MASTER SLAVE SYSTEM (MEDICAL MANIPULATOR)
2: MASTER INPUT PART (MANIPULATION PART)
23: FOOT SWITCH (MODE-INPUT PART)
50, 50A, 150: INSERTION PART
51: EXTERNAL CYLINDRICAL TUBE
52: BENDING PART
61, 161: ARM
65, 165, 265: ARM PART
71,271: TREATMENT TOOL
73: TREATMENT PART
92: COMPUTATION PART (CONTROL PART)

## Claims

1. A medical manipulator comprising:
an insertion part which includes an external cylindrical tube that has a bendingly manipulable bending part and that has flexibility;
a manipulation part which performs a manipulation input for manipulating the insertion part;
an arm part which is provided on a distal end part of the insertion part and has a bendingly manipulable arm and a treatment part that performs a procedure;
a mode-input part which is provided on the manipulation part and is capable of inputting a mode that is selected among a plurality of modes that are combinations of states of the bending part and the arm part; and
a control part that selectively controls the bending part and the arm part such that the bending part and the arm part are controlled to enter one of two states consisting of a locked state in which the bending part and the arm part are locked in a predetermined state in which the manipulation input is not accepted, and a non-locked state, on the basis of the manipulation input and a mode input to the mode-input part.

2. The medical manipulator according to Claim 1,
wherein at least one of the locked states of the arm part is a retracted state in which the arm part is arranged within a width of the external cylindrical tube.

3. The medical manipulator according to Claim 2,
wherein at least one of the non-locked states is a manipulable state in which the bending part or the arm part is operated according to the manipulation input.

4. The medical manipulator according to Claim 3,
wherein at least one of the plurality of modes is an insertion mode suitable for inserting the insertion part into a body, and
in the insertion mode, the bending part is brought into the manipulable state and the arm part is brought into the retracted state.

5. The medical manipulator according to Claim 4,
wherein a first rigid length of the insertion part on a distal end side in the retracted state is smaller than a maximum value of a second rigid length in a procedure.

6. The medical manipulator according to Claim 4,
wherein at least one of the plurality of modes is a treatment mode suitable for performing a procedure using the treatment part, and in the treatment mode, the bending part is brought into the locked state and the arm part is brought into the manipulable state.

7. The medical manipulator according to Claim 4,
wherein at least one of the plurality of modes is a treatment mode suitable for performing a procedure using the treatment part, and
in the treatment mode, the bending part and the arm part are brought into the manipulable state.

8. The medical manipulator according to any one of Claims 4 to 7,
wherein at least one of the plurality of modes is an urgent extraction mode suitable for rapidly extracting the insertion part out of the body, and
in the urgent extraction mode, the bending part and the arm part are brought into a free state which is one of the non-locked states and in which an operation in response to the manipulation input is not performed and deformation by an external force is allowed.

9. The medical manipulator according to Claim 6 or 7,
wherein the arm part has the arm fixed to the distal end part of the external cylindrical tube, and a treatment tool having the treatment part on a distal end part of the treatment part and being inserted through the external cylindrical tube and the arm, and
wherein a protruding amount of the treatment part from the arm in the insertion mode is smaller than a protruding amount of the treatment part from the arm in the treatment mode.
